# EUROPÄISCHE PATENTSCHRIFT

(11) **EP 2 763 647 B1**
(45) Veröffentlichungstag und Bekanntmachung des Hinweises auf die Patenterteilung: **25.12.2019**
(21) Anmeldenummer: 13759979.1
(22) Anmeldetag: 04.07.2013
(51) Int. Cl.: A61K 6/00

(54) **HAFTZUBEREITUNG FÜR KIEFERPROTHESEN**
ADHESIVE PREPARATION FOR MANDIBULAR PROSTHESES
PRÉPARATION ADHÉSIVE POUR PROTHÈSES DE LA MÂCHOIRE

(30) Priorität: 03.01.2013 WO PCT/EP2013/000007
(43) Veröffentlichungstag der Anmeldung: 13.08.2014
(73) Patentinhaber: Tripp GmbH & Co. KG, 77728 Oppenau (DE)
(72) Erfinder: HAYAG, Hans, 77654 Offenburg (DE); HENKEL, Lutz, 79415 Bad Bellingen / Hertingen (DE); SÖLLNER-TRIPP, Hanspeter, 77704 Oberkirch (DE)
(74) Vertreter: Lichti - Patentanwälte Partnerschaft mbB
(86) Internationale Anmeldenummer: PCT/EP2013/002573
(87) Internationale Veröffentlichungsnummer: WO 2014/106516

(56) Entgegenhaltungen:
- WO-A1-2011/088988
- WO-A1-2011/088988
- US-A- 4 542 168
- US-A- 4 542 168
- US-A- 5 561 177
- US-A- 5 561 177

## Beschreibung

Die Erfindung betrifft eine Kieferprothesen-Haftzubereitung.

Eine Haftzubereitung für Kieferprothesen ist aus der DE 10 2005 031 771 bekannt. Die dortige Haftzubereitung enthält dickflüssiges Paraffin in einem Anteil von 25 bis 35 Gew.-% und/oder weiße Vaseline in einem Anteil von 10 bis 20 Gew.-% als Cremebasis, ein Carbomer (Polyacrylsäure) als Haftmittel mit einem Anteil von 1 bis 15 Gew.-% sowie als Haftmittel eine oder mindestens zwei Carboxymethylcellulose/n oder ein Alkalimetall- oder Erdalkalimetallsalz derselben mit einem Anteil von 15 bis 50 Gew.-% sowie hochdisperse Kieselsäure mit 0,2 bis 2,0 Gew.-% als Füllstoff.

Handelsübliche Haftcremes verwenden Paraffin/Vaseline, um eine geschmeidige Zubereitung zu erzielen. Bei Prothesenträgern können Irritationen der Rachenpartien auftreten. Die im Markt gängigen Prothesen-Haftcremes nutzen zum größten Teil auch den Rohstoff Gantrez (Calcium/Sodium PVM/MA Copolymer) der Firma ISP, bezüglich dessen die Gefahr von möglichen Rohstoffverknappungen, Rohstoffabhängigkeiten und zeitlichen Verzögerungen der Rohstoffverfügbarkeit gegeben ist.

Es wurde daher in der Praxis schon vorgeschlagen, als Trägersubstanz statt Paraffin oder Vaseline Olivenöl zu verwenden. Die bekannte Haftcreme weist allerdings lediglich eine Haftfähigkeit bestenfalls im unteren vertretbaren Bereich auf.

Will man Paraffin oder Vaseline durch ein Pflanzenöl ersetzen, so können die übrigen Komponenten der Ausgangs-Haftzubereitung nicht als solche beibehalten werden. Aufgrund der völlig anderen Konsistenz von Olivenöl gegenüber Vaseline oder Paraffin ergibt sich dann keine akzeptable Haftzubereitung.

So sind aus der US 5,561,177 und der WO 2011/088988 jeweils Haftzubereitungen auf der Basis von Pflanzenölen mit grundsätzlich ähnlichen Anteilen und ähnlichen Anteilen an strukturbildendem Stabilisator, aber Haftmittelanteilen von 20 bis 25 Gew.-% in Form von Alkylvinyl/ Maleinsäureanhydrid-Copolymer bekannt.

Die US 4,542,168 schlägt eine zwar eine Haftzubereitung mit Carbopol als Haftmittel vor, das nach einem einzigen Ausführungsbeispiel mit 10 Gew.-% angegeben wird, während bei den anderen Ausführungsbeispielen der Anteil wesentlich höher liegt, insbesondere sein Schwergewicht bei 25 bis 35 Gew.-% hat. Darüber hinaus ist hier zu beachten, dass das Haftmittel in einer Vorstufe als monovalentes Kation und lediglich teilverlinkt eingesetzt wird und erst in einer weiteren Stufe durch Neutralisation zum eigentlichen Haftmittel umgewandelt wird. Dieser Prozess ist nur schwierig steuerbar, insbesondere hinsichtlich des tatsächlichen Anteils des Haftmittels in der fertigen Zusammensetzung.

Bei der Schaffung einer Haftzubereitung ist insbesondere den nicht komplementären Forderungen hinsichtlich Haftvermögen, Konsistenz und Beständigkeit Rechnung zu tragen.

Der Erfindung liegt daher die Aufgabe zugrunde, unter Vermeidung der genannten Vorteile eine Haftzubereitung für Kieferprothesen zu schaffen, die unter Verzicht auf Paraffin und/oder Vaseline als Grund- oder Cremesubstanz dennoch nicht nur gute objektive, sondern auch subjektive Hafteigenschaften aufweisen und so dem Einsatz als Haftzubereitung geeignet ist und insbesondere auch eine homogene Konsistenz und hohe zeitliche Beständigkeit hat.

Erfindungsgemäß wird die genannte Aufgabe mit einer Haftzubereitung der eingangs genannten Art gelöst, die die kennzeichnenden Merkmale des Anspruchs 1 aufweist.

Die Haftcreme besteht ausschließlich aus diesen Komponenten. Dabei liegt die Summe von Pflanzenöl und Stabilisator unter 90 Gew.-%.

Der Füllstoff bzw. dessen in zumindest bestimmender Hauptkomponente Siliziumdioxid (Silica) ist hoch porös. Der Füllstoff und/oder das Siliziumdioxid weist insbesondere eine Dichte von 1,9 bis 2,1 g/cm³ auf. Hierdurch wird eine gute Durchmischung und Verbindung der weiteren Komponenten erreicht, was dazu beiträgt, dass trotz geringen Haftmittelanteils gute objektive Hafteigenschaften erreicht werden und sich so die Zusammensetzung für den Nutzer auch angenehm anfühlt. Während üblicherweise Siliziumdioxid als gebranntes oder pyrogenes Siliziumdioxid hergestellt wird, ist nach der Erfindung vorgesehen, dass der Füllstoff aus einer Lösung ausgefälltes Siliziumdioxid enthält. Gemäß weiterer bevorzugter Ausgestaltung liegt die Teilchengröße im Bereich von 1 bis 40 µm. Teilchengröße bezeichnet hier nicht die Primärpartikelgröße, die bei 5-100 nm liegt, sondern die Größe der durch Agglomeration der Primärpartikel gebildeten Teilchen, oder des Agglomerat-Teilchens oder die Größe des Agglomerats. Die durchschnittliche Porengröße vorzugsweise > 30 nm ist. Während vorzugsweise die Oberfläche der Teilchen im Bereich von 5 bis 100 m²/g liegt, liegt dieser Wert höchst vorzugsweise < als 50m²/g.

Als Ersatz für Paraffin/Vaseline gewährleisten Pflanzenöle die Geschmeidigkeit der Zubereitung. Mit dem Einsatz von Pflanzenölen kann aufgrund der chemischen Zusammensetzung ein entzündungshemmender Effekt bei entsprechenden Irritationen erzielt werden. Auch das Haftmittel trägt zur Geschmeidigkeit bei. Der Füllstoff bewirkt die erforderliche Verdickung und regelt die Viskosität.

Es wurde festgestellt, dass bevorzugt als Pflanzenöl neben Olivenöl insbesondere auch Johanniskraut-, Mandel-, Raps-, Soja-, Sonnenblumen-, Traubenkern- und/oder Weizenkeimöl in Frage kommen.

Das Haftmittel enthält Polyacrylsäure und zwar insbesondere als ausschließliches Haftmittel. Dabei wird bevorzugt das Haftmittel mit einer dynamischen Viskosität gleich oder größer 30000 mPas eingesetzt. Als konkrete Polyacrylsäure oder Carbomere kommen insbesondere unter den Handelsnamen Carbopol 971P NF und Carbopol 974P NF vertretene Carbomere in Frage. Durch dieses Haftmittel kann das üblicherweise bei Haftzusammensetzung eingesetzte Haftmittel (Methylvenylether), Maleinsäureanhydrid(-Copolymer), das als bedenklich angesehenes Zink enthält, vermieden werden.

Bevorzugte Ausgestaltungen der Erfindung sehen vor, dass die Dichte des Füllstoffs, vorzugsweise zumindest des ihn im Wesentlichen konstituierenden Siliziumdioxids zwischen 0,08 - 0,23 g/ cm³, vorzugsweise zwischen 0,19 und 0,21 g/cm³ beträgt und/oder dass die Teilchengröße im Bereich von 1 bis 40 µm liegt, wobei die durchschnittliche Porengröße vorzugsweise > 30 nm ist.

Während der Füllstoff ausschließlich aus Siliziumoxid (Silica) bestehen kann, weist er in bevorzugter Ausgestaltung einen geringen Anteil von weniger als 0,1 Gew.-%, vorzugsweise aber mindestens 0,001 Gew.-%, höchst vorzugsweise mindestens 0,01 Gew.-% Styren-Isopren-Copolymer und/oder weniger als 0,1 Gew.-%, vorzugsweise mindestens 0,001 Gew.-%, höchst vorzugsweise mindestens 0,01 Gew.-% Aluminiumstearat auf.

Während die oben genannten Zusammensetzungen mit den dort gegebenen Gewichtsanteilen geeignete Kieferprothesen-Haftzubereitungen gewährleisten, ist in bevorzugter Ausgestaltung vorgesehen, dass dem Pflanzenölanteil 40 bis 50 Gew.-%, der die Struktur bildende Stabilisator 40 bis 50 Gew.-%, das Haftmittel 8 bis 13 Gew.-% und/oder der Füllstoff 3 bis 6 Gew.-% ausmachen, wobei die Summe sämtlicher Bestandteile jeweils 100 Gew.-% beträgt.

Durch die Erfindung wird eine Haftzubereitung mit einer plastischen, weichen Konsistenz geschaffen, die weder zu steif noch zu fließfähig ist. Die Haftzubereitung hat darüber hinaus eine geeignete Haftkraft oberhalb eines unteren vertretbaren Wertes von 40 Newton, ohne dass die Gefahr einer zu großen Haftkraft von über 150, insbesondere über 180 Newton gegeben ist.

Darüber hinaus weist die erfindungsgemäße Haftzubereitung eine hohe - zeitliche - Beständigkeit auf und zersetzt sich auch bei langer Lagerzeit nicht.

Weitere Vorteile und Merkmale der Erfindung ergeben sich aus den Ansprüchen und aus der nachfolgenden Beschreibung, in der Ausführungsbeispiele der erfindungsgemäßen Haftzusammensetzung erläutert sind. Dabei zeigt:

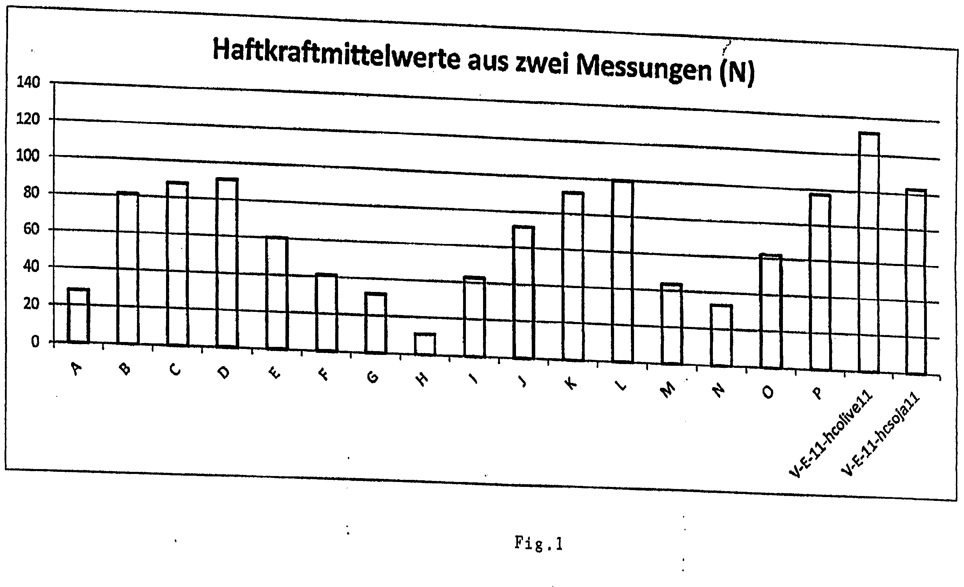

- Fig. 1: Haftkraftwerte für verschiedene Haftzubereitungen.

Die Fig. 1 zeigt Haftkraft-Mittelwerte aus zwei Messungen in Newton für eine Vielzahl im Markt befindlicher Haftkraftzubereitungen, die mit A bis P bezeichnet sind sowie zwei erfindungsgemäße Haftkraftzubereitungen einerseits auf der Basis von Olivenöl, andererseits auf der Basis von Sojaöl, deren genaue Zusammensetzung sich aus den Tabellen 1 bis 3 ergibt.

Bei der Haftzusammensetzung H handelt es sich um die aus der Praxis bekannte Zusammensetzung auf der Grundlage von Olivenöl und ohne Erdöl basierten Grundsubstanzen, wie Paraffin oder Vaseline, die bei sämtlichen anderen Haftzusammensetzungen A bis G, I bis P gegeben sind.

Es zeigt sich, dass, während die bekannte Olivenöl basierte Haftzusammensetzung gegenüber ausreichend vertretbaren Haftkraftwerten von über 40 oder 50 Newton eine demgegenüber äußerst geringe Haftkraft von weniger als 20 Newton aufweist, die ebenfalls auf Pflanzenölen beruhenden erfindungsgemäßen Haftkraftzusammensetzung VE-E-11-hcsoja11 optimale Haftkraftwerte im Bereich von 100 Newton zeigt, wobei, wie gesagt, Haftkraftwerte von über 50 Newton durchaus noch vertretbar sind, die allerdings bisher lediglich durch Paraffin- oder Vaseline- basierte Haftkraftzusammensetzungen erzielt werden.

Bei dem Füllstoff der Tabellen handelt es sich durchgehend um Siliziumoxid basierten Füllstoff mit 99 % Siliziumoxid (Silica) und geringen Anteilen von Styren-Isopren-Copolymer und Aluminiumstearat von jeweils weniger als 0,1 Gew.-%. Soweit in den Tabellen jeweils keine spezifischen Angaben enthalten sind, gelten die im Kopf der Tabellen angegebenen Komponentenbezeichnungen und Anteile. Abweichende Komponenten sind in den einzelnen Feldern der Tabelle gegebenenfalls selbst angegeben, ebenso wie abweichende Prozentzahlen der im Kopf angegebenen Komponenten.

Die den als Anhang beigefügten Tabellen 1 bis 3 zugrundeliegenden Haftkraft- und Konsistenzmessungen werden in folgender Weise durchgeführt, letztere analog DIN 10331/ISO 16305:
Die Haftkraftmessungen werden mit dem Strukturmessgerät TA.XT plus der Firma Stable Micro Systems Ltd., Godalming, Surrey, Großbritannien, vorgenommen. Zur Messung wurde von Haftcreme 0,75 g (+/-0,01g) mit einer Probentemperatur von 25°C +/- 1°C punktförmig auf eine ungravierte Seite des Probenträgers aufgebracht. Die Vor- und Testgeschwindigkeit betragen jeweils 0,5 mm/sec, die Rückgeschwindigkeit 10,0 mm/sec. Die Anpresskraft betrug 1000,0 g, der Auslösewert 5,0 g. Es wurden 500 Messpunkte pro Messung erfasst.

Die Konsistenzmessung erfolgte derart, dass auf einer Glasplatte in der Mitte 1,0 g der auf 20°C temperierten Prüfsubstanz punktförmig aufgetragen werden. Die Glasplatte mit der Prüfsubstanz wurde für 10 bis 15 min auf das auf 20°C temperierte Extensometer gelegt, bevor eine weitere Glasplatte (Durchmesser = 11,5 cm) mit einem Gewicht von 47,85 g aufgelegt wurde. Dann wurde die obere Glasplatte mit dem Prüfgewicht der Apparatur von 331,3 g beschwert (Gesamtmasse 379,15 g). Der Durchmesser des entstandenen Kreises oder der Ellipse wurde nach 15 min. bestimmt. Die Spalten der Tabellen geben oben Anteilswerte der Temperaturen an. Sind diese bei einer Zusammensetzung exakt gegeben wird dies mit einem "x" vermerkt. Weicht der genaue Wert bei gleicher Größenordnung ab, ist der genaue Wert angegeben.

In Tabelle 1 ist in Zeile 1 eine äußerst bevorzugte erfindungsgemäße Rezeptur V-E-11-hcsoja-11 dargestellt mit 42 Gew.-% Sojaöl, 9 Gew.-% Polyacrylsäure als Haftmittel, wie es unter der Bezeichnung Carbopol 971P NF von der Lubrizol Corporation, Wickliffe, Ohio, USA vertrieben wird, 44 % beispielsweise von der Dow Deutschland Anlagengesellschaft mbH, Schwalbach unter der Bezeichnung WALOCEL vertriebener Carbonmethylcellulose als Struktur bildender Stabilisator und 5 Gew.-% Füllstoff. Diese Haftzubereitung weist eine optimale - weiche - Konsistenz von 4,4 bis 4,5 cm mit einer, wie die Fig. 1 zeigt, Haftkraft von 92 Newton auf. Während die Rezepturen 4 bis 6 der Tabelle 1 mit Konsistenzen zwischen 3,5 und 3,8 cm eine ebenfalls akzeptable Haftkraft von 63 bis 76 Newton aufweisen, zeigt sich, dass bei einer stärkeren Reduzierung des Pflanzenölanteils unter gleichzeitiger Erhöhung des Carboxymethylcelluloseanteils auf deutlich über 50 % die Konsistenz der Haftzubereitung zu steif oder zu fest ist (Rezeptur 2 der Tabelle 1) und dies auch nicht durch Reduzierung des Füllstoffs kompensiert werden kann (Rezeptur 3 der Tabelle 1).

Die Tabelle 2 zeigt, dass der Struktur bildende Stabilisator Carboxymethylcellulose (Walocel) grundsätzlich auch durch den Struktur bildenden Stabilisator Xanthan vollständig - oder auch teilweise - substituiert werden kann, wobei die Haftkraft gegenüber Carboxymethylcellulose zwar geringfügig abnimmt, aber durchaus auch deutlich über der unteren Grenze von 40 Newton liegt. Die Tabelle zeigt daher auch, dass Haftmittel mit deutlich höherer Viskosität als 29000 mPa eingesetzt werden können und durchaus - sehr - gute Haftwerte bei geeigneter Konsistenz erreicht werden.

Erfindungsgemäße Haftzubereitungen weisen einen körperverträglichen, insbesondere auch schleimhautverträglichen leicht sauren pH-Wert von mehr als 5 auf.

Überraschenderweise zeigen die Rezepturen 4 und 5 der Tabelle 3, dass der Ersatz eines geeigneten Haftmittels - wie bei den anderen Zusammensetzungen eingesetzt - durch ein als Struktur gebender Stabilisator geeignetes Mittel, wie eben Keldent, zu einem dramatischen Abfall der Haftkraft führt, abgesehen davon, dass die Haftzubereitung darüber hinaus auch zu steif wird.

### Anhang 1

**Tabelle 1**

| **NEUE HAFTCREME - TEST-REIHEN 2011** | | | | | | | | | | |
|---|---|---|---|---|---|---|---|---|---|---|
| | | **Natürliches Öl** | | **Carbopol 971P NF** | | **CMC** | | **Füllstoff** | | **PHYSIKALISCHE EIGENSCHAFTEN** |
| | | 30% | 45% | 5% | 15% | 30% | 50% | 3% | 6% | |
| 1 | **REZEPTUR V-E-11-hcsoja-11** | | **42%** | **9%** | | | **44%** | | **5%** | **Konsistenz = 4,4 / 4,5 cm Haftkraft = 92N** |
| 2 | V-E-11hcsoja12-a | x | | 9% | | | 56% | | 5% | =>zu fest, Geschmeidigkeit fehlt! |
| 3 | V-E-11-hcsoja12 | x | | 9% | | | 58% | x | | =>zu fest, Geschmeidigkeit fehlt! |
| 4 | V-E-11-hcsoja13 | | x | 9% | | | 40% | | x | Konsistenz = 3,8 / 3,8 cm Haftkraft = 76N |
| 5 | V-E-11-hcsoja14 | | x | x | | | 44% | | x | Konsistenz = 3,5 / 3,6 cm Haftkraft = 63N |
| 6 | V-E-11-hcsoja15 | | x | | x | 34% | | | x | Konsistenz = 3,5 / 3,5 cm Haftkraft = 75N |

### Anhang 2

**Tabelle 2**

| **NEUE HAFTCREME - TEST-REIHEN 2011** | | | | | | |
|---|---|---|---|---|---|---|
| | | **Natürliches Öl** | **Carbopol 974P NF** | **Struktur** | **Füllstoff** | **PHYSIKALISCHE EIGENSCHAFTEN** |
| | | 45% | 5% | 45% | 6% | |
| 1 | **REZEPTUR V-E-11-hcsoja-11** | **42%** | **9% Carbopol 971P NF** | **44% Walocel 60.000** | **5%** | **Konsistenz = 4,4 / 4,5 cm Haftkraft = 92N** |
| 2 | V-E-11hccarb974-2 | 45% | 15% | 35% Walocel 60.000 | 5% | Konsistenz = 3,6 / 3,6 cm Haftkraft = 78N |
| 3 | V-E-11hccarb974-2 | 45% | 15% | 38% Keldent | 5% | Konsistenz = cm Haftkraft = 77N |
| 4 | V-E-11hccarb974-2 | 42% | 9% | 44% Keldent | 5% | Konsistenz = 3,6 / 3,6 cm Haftkraft = 69N |

### Anhang 3

**Tabelle 3**

| **NEUE HAFTCREME - TEST-REIHEN 2011** | | | | | | |
|---|---|---|---|---|---|---|
| | | **Natürliches Öl** | **Carbopol 971P NF**** | **CMC [oder] HEC [oder] Xanthan Gum***** | **99% Silica**** < 0,1% Syrene/Isoprene Copolymer < 0,1% Aluminium Stearates** | **PHYSIKALISCHE EIGENSCHAFTEN** |
| | | 45% | 5% | 45% | 6% | |
| 1 | **PATENTREZEPTUR V-E-11-hcsoja-11** | **42%** | **9%** | **44% Walocel 60.000** | **5%** | **Konsistenz = 4,4 / 4,5 cm Haftkraft = 92N** |
| 2 | V-E-11hckel-1 | 42% | 9% | 44% Keldent | 5% | Konsistenz = 4,0 / 4,0 cm Haftkraft = 72N |
| 3 | V-E-11hckel-2 | 42% | 9% | 44% Keltrol CG | 5% | Konsistenz = 2,9 / 3,0 cm Haftkraft = 25N |
| 4 | V-E-11hckel-3 | 42% | 9% | 53% Keldent | 5% | **Haftkraft = 1,3N** =>zu fest, Geschmeidigkeit fehlt! |
| 5 | V-E-11hckel-4 | 42% | x | 44% Walocel 60.000 | 5% | **Haftkraft** = **0,6N** =>zu fest, Geschmeidigkeit fehlt! |
| 6 | V-E-11-honat-1 | 42% | x | 44% Natrosol HX | 5% | Haftkraft = 39N => zu fest, grießelig, Geschmeidigkeit fehlt! |

## Patentansprüche

1. Kieferprothesen-Haftzubereitung mit
- 35 bis 50 Gew.-% Pflanzenöl,
- 25 bis 50 Gew.-% Struktur bildendem Stabilisator Carboxymethylcellulose (CMC) oder dem Xanthan Keldent®
- 5 bis 15 Gew.-% Haftmittel, und
- 3 bis 6 Gew.-% Siliziumdioxid enthaltendem Füllstoff, wobei die Summe sämtlicher Bestandteile 100 Gew.-% beträgt,
wobei das Haftmittel Polyacrylsäure enthält, und der Füllstoff aus einer Lösung ausgefälltes Siliziumdioxid mit einer Dichte zwischen 0,08 und 0,23 g/cm³ enthält und derart hochporös ist.

2. Haftzubereitung nach Anspruch 1, **dadurch gekennzeichnet, dass** das Pflanzenöl Johanniskraut-, Mandel-, Oliven-, Raps-, Soja-, Sonnenblumen-, Traubenkern-, und/oder Weizenkeimöl ist.

3. Haftzubereitung nach Anspruch 1 oder 2, **dadurch gekennzeichnet, dass** die Dichte des Füllstoffs, vorzugsweise zumindest des ihn im Wesentlichen konstituierenden Siliziumdioxids zwischen 0,19 und 0,21 g/cm³ beträgt.

4. Haftzubereitung nach einem der vorangehenden Ansprüche, **dadurch gekennzeichnet, dass** die Teilchengröße im Bereich von 1 bis 40 µm liegt, wobei die durchschnittliche Porengröße vorzugsweise > 30 nm ist.

5. Haftzubereitung nach einem der vorangehenden Ansprüche, **dadurch gekennzeichnet, dass** der Füllstoff weniger als 0,1 Gew.-%, höchst vorzugsweise mindestens 0,001 Gew.-%, vorzugsweise mindestens 0,01 Gew.-% Styren-Isopren-Copolymer bezogen auf die Füllstoffmasse enthält.

6. Haftzubereitung nach einem der vorangehenden Ansprüche, **dadurch gekennzeichnet, dass** der Füllstoff weniger als 0,1 Gew.-%, höchst vorzugsweise mindestens 0,001 Gew.-%, vorzugsweise mindestens 0,01 Gew.-% Aluminiumstearat bezogen auf die Masse des Füllstoffs enthält.

7. Haftzubereitung nach einem der vorangehenden Ansprüche, **gekennzeichnet durch** eine Haftkraft zwischen 40 und 150 N, vorzugsweise zwischen 70 und 110 N.

8. Haftzubereitung nach einem der vorangehenden Ansprüche, **gekennzeichnet durch** einen Pflanzenölanteil von 40 bis 45 Gew.-%.

9. Haftzubereitung nach einem der vorangehenden Ansprüche, **gekennzeichnet durch** 40 bis 50 Gew.-% Struktur bildenden Stabilisator.

10. Haftzubereitung nach einem der vorangehenden Ansprüche, **gekennzeichnet durch** 5 bis weniger als 10 Gew.-% vorzugsweise höchstens 9% Haftmittel.

11. Haftzubereitung nach einem der vorangehenden Ansprüche, **gekennzeichnet durch** 40 bis 45 Gew.-% Pflanzenöl, 41 bis 46 Gew.-% Struktur bildender Stabilisator, 7 bis weniger als 10 Gew.-% vorzugsweise höchstens 9 Gew.-% Polyacrylsäure und 4 bis 6 Gew.-% Siliziumoxid enthaltendem Füllstoff.

## Claims

1. Adhesive preparation for mandibular prostheses comprising:
- 35 to 50% by weight of vegetable oil,
- 25 to 50% by weight of structure-forming stabilizer carboxymethyl cellulose (CMC) or of the xanthan Keldent®
- 5 to 15% by weight of bonding agent and
- 3 to 6% by weight of silica-containing filler, wherein the sum of all the constituents is 100% by weight,
wherein the bonding agent comprise polyacrylic acid and the filler comprise silica precipitated from solution and having a density between 0.08 and 0.23 g/cm³ and is thus highly porous.

2. Adhesive preparation according to Claim 1, **characterized in that** the vegetable oil is St. John's wort oil, almond oil, olive oil, rapeseed oil, soybean oil, sunflower oil, grape seed oil, and/or wheatgerm oil.

3. Adhesive preparation according to Claim 1 or 2, **characterized in that** the density of the filler, preferably at least of the silica of which it is largely comprised, is between 0.19 and 0.21 g/cm³.

4. Adhesive preparation according to any of the preceding claims, **characterized in that** the particle size is in the range from 1 to 40 µm, wherein the mean pore size is preferably > 30 nm.

5. Adhesive preparation according to any of the preceding claims, **characterized in that** the filler comprises less than 0.1% by weight, most preferably not less than 0.001% by weight, preferably not less than 0.01% by weight of styrene-isoprene copolymer, based on the mass of the filler.

6. Adhesive preparation according to any of the preceding claims, **characterized in that** the filler comprises less than 0.1% by weight, most preferably not less than 0.001% by weight, preferably not less than 0.01% by weight of aluminium stearate, based on the mass of filler.

7. Adhesive preparation according to any of the preceding claims, **characterized by** a bonding strength of between 40 and 150 N, preferably between 70 and 110 N.

8. Adhesive preparation according to any of the preceding claims, **characterized by** a vegetable oil content of from 40 to 45% by weight.

9. Adhesive preparation according to any of the preceding claims, **characterized by** 40 to 50% by weight of structure-forming stabilizer.

10. Adhesive preparation according to any of the preceding claims, **characterized by** 5 to less than 10% by weight, preferably not more than 9%, of bonding agent.

11. Adhesive preparation according to any of the preceding claims, **characterized by** 40 to 45% by weight of vegetable oil, 41 to 46% by weight of structure-forming stabilizer, 7 to less than 10% by weight, preferably not more than 9% by weight, of polyacrylic acid, and 4 to 6% by weight of silica-containing filler.

## Revendications

1. préparation adhésive pour prothèses de mâchoire avec :
- 35 à 50 % en poids d'huile végétale ;
- 25 à 50 % en poids de stabilisateur formant la structure de type carboxyméthylcellulose (CMC) ou de xanthane Keldent® ;
- 5 à 15 % en poids d'adhésif ; et
- 3 à 6 % en poids de dioxyde de silicium contenant de la charge, la somme de l'ensemble des composants étant de 100 % en poids ;
l'adhésif contient de l'acide polyacrylique et une charge d'une solution de dioxyde de silicium précipité de masse volumique comprise entre 0,08 et 0,23 g/cm³, ce qui le rend hautement poreux.

2. Préparation adhésive selon la revendication 1, **caractérisée en ce que** l'huile végétale est de l'huile de millepertuis, d'amande, d'olive, de colza, de soja, de tournesol, de pépin de raisin et/ou de germe de blé.

3. Préparation adhésive selon la revendication 1 ou 2, **caractérisée en ce que** la masse volumique de la charge et de préférence au moins du dioxyde de silicium la constituant est pour l'essentiel comprise entre 0,19 et 0,21 g/cm³.

4. Préparation adhésive selon l'une quelconque des revendications précédentes, **caractérisée en ce que** la taille de particule se situe dans la plage de 1 à 40 µm, la taille moyenne de pore étant de préférence > 30 µm.

5. Préparation adhésive selon l'une quelconque des revendications précédentes, **caractérisée en ce que** la charge contient moins de 0,1 % en poids, de façon préférée entre toutes au moins 0,001 % en poids, de préférence au moins 0,01 % en poids de copolymère styrène-isopropène par rapport à la masse de la charge.

6. Préparation adhésive selon l'une quelconque des revendications précédentes, **caractérisée en ce que** la charge contient moins de 0,1 % en poids, de façon préférée entre toutes au moins 0,001 % en poids, de préférence au moins 0,01 % en poids de stéarate d'aluminium par rapport à la masse de la charge.

7. Préparation adhésive selon l'une quelconque des revendications précédentes, **caractérisée par** une force d'adhérence comprise entre 40 et 150 N, de préférence entre 70 et 110 N.

8. Préparation adhésive selon l'une quelconque des revendications précédentes, **caractérisée par** une teneur en huile végétale de 40 à 45 % en poids.

9. Préparation adhésive selon l'une quelconque des revendications précédentes, **caractérisée par** 40 à 50 % en poids de stabilisateur formant la structure.

10. Préparation adhésive selon l'une quelconque des revendications précédentes, **caractérisée par** 5 à moins de 10 % en poids, de préférence de tout au plus 9 % d'adhésif.

11. Préparation adhésive selon l'une quelconque des revendications précédentes, **caractérisée par** 40 à 45 % en poids d'huile végétale, 41 à 46 % en poids de stabilisateur formant la structure, 7 à moins de 10 % en poids, de préférence de tout au plus 9 % en poids d'acide polyacrylique et 4 à 6 % en poids de charge contenant de l'oxyde de silicium.
